# EUROPÄISCHE PATENTSCHRIFT

(11) **EP 0 749 412 B1**
(45) Veröffentlichungstag und Bekanntmachung des Hinweises auf die Patenterteilung: **27.01.1999**
(21) Anmeldenummer: 95912183.1
(22) Anmeldetag: 02.03.1995
(51) Int. Cl.: C07C 233/36, C07C 233/38, C07C 59/06, C07C 59/08, C11D 1/90, C11D 1/94

(54) **NIEDRIGVISKOSE WÄSSRIGE KONZENTRATE VON BETAINTENSIDEN**
LOW-VISCOSITY AQUEOUS BETAINE SURFACTANT CONCENTRATES
CONCENTRES AQUEUX DE FAIBLE VISCOSITE D'AGENTS TENSIOACTIFS DE BETAINE

(30) Priorität: 11.03.1994 DE 4408183
(43) Veröffentlichungstag der Anmeldung: 27.12.1996
(73) Patentinhaber: Henkel Kommanditgesellschaft auf Aktien, 40191 Düsseldorf (DE)
(72) Erfinder: BIGORRA LLOSAS, Joaquim, E-08203 Sabadell (ES); PI SUBIRAMA, Rafael, E-08400 Granollers (ES)
(86) Internationale Anmeldenummer: EP9500755
(87) Internationale Veröffentlichungsnummer: WO9524376

(56) Entgegenhaltungen:
- EP-A- 0 353 580
- EP-A- 0 492 228
- EP-A- 0 560 114
- DE-C- 4 207 386

## Beschreibung

### Gebiet der Erfindung

Die Erfindung betrifft niedrigviskose wäßrige Konzentrate von Betaintensiden mit einem Gehalt an ausgewählten Hydroxycarbonsäuren, ein Verfahren zu ihrer Herstellung sowie die Verwendung von Hydroxycarbonsäuren als Verflüssigungsmittel für wäßrige Betainkonzentrate.

### Stand der Technik

Betaine bzw, amphotere Tenside sind ausgesprochen hautverträglich und weisen ausgezeichnete Reinigungseigenschaften auf. Sie eignen sich daher in besonderer Weise zur Konfektionierung einer Vielzahl von oberflächenaktiven Produkten. Zu ihrer Herstellung geht man im einfachsten Fall von tertiären Aminen aus, die mit Natriumchloracetat zu Alkylbetainen umgesetzt werden. Die Umsetzung von Fettsäureaminoamiden oder Imidazolinen mit Natriumchloracetat führt zur Bildung von amphoteren Tensiden vom Typ der Glycinate; wird als Alkylierungsmittel Acrylsäureester eingesetzt, bilden sich Aminopropionate. Verbindungen der genannten Art sind in einer Vielzahl von Übersichtsartikeln beschrieben, von denen an dieser Stelle nur **Parf.Cosm.Arom. 70, 67 (1986), HAPPI, 70, (Nov.1986)** und **Soap Cosm.Chem.Spec. 46, (Apr.1990)** genannt sein sollen.

Ein besonderes Anliegen bei der Herstellung der Betaine bzw. amphoteren Tenside besteht darin, möglichst reine und somit dermatologisch und toxikologisch unbedenkliche Produkte zur Verfügung zu stellen. Unerwünscht sind beispielsweise Spuren von freien Aminen, Chloressigsäure und insbesondere Dichloressigsäure in den Tensiden. Auch Konservierungsstoffe, die die Betaine bzw. amphoteren Tenside vor mikrobiellem Befall schützen sollen, sind häufig nicht erwünscht, so daß ein weiteres Bedürfnis nach Produkten besteht, die auch ohne Zusatz von Hilfsstoffen gegenüber Keimbefall stabilisiert sind. Eine dritte Aufgabe der Erfindung besteht schließlich darin, möglichst hellfarbige Produkte mit einem hohen Feststoffgehalt, vorzugsweise im Bereich von 40 bis 50 Gew.-% zur Verfügung zu stellen. Aus dem Stand der Technik sind bereits eine Reihe von Druckschriften bekannt, die Teillösungen für die kumulierte Aufgabenstellung anbieten.

So wird beispielsweise in der **DE-A1 3939264** (Henkel) vorgeschlagen, den Gehalt an Chloressigsäure in amphoteren Tensiden durch eine nachträgliche Behandlung der wäßrigen Lösungen mit Ammoniak, Aminosäuren oder Oligopeptiden zu verringern. Aus der **DE-OS 2926479** (Th.Goldschmidt) ist ein Verfahren bekannt, bei dem man die Quaternierung im pH-Bereich von 7,5 bis 10,5 durchführt und so den Restgehalt an freiem Alkylierungsmittel minimiert. In die gleiche Richtung weist die Lehre der **DE-A 2063424** (Rewo), die die pH-Regulierung für die Alkylierung von Imidazolinen beschreibt. Ferner wird in der **DE-C 3726322** (Th.Goldschmidt) ein Verfahren zur Nachbehandlung von Betainen beschrieben, bei dem man den Stoffen Mineralsäuren in solchen Mengen zusetzt, daß der pH-Wert der Lösung 1 bis 4,5 beträgt. Auf den Gehalt an Dichloressigsäure haben diese Verfahren jedoch keinen Einfluß. In der **DE-A1 4205880** (Th.Goldschmidt) wird zur Minimierung von chlorierten Verunreinigungen vorgeschlagen, die Betaine in wäßriger Lösung bei einer Temperatur im Bereich von 115 bis 180°C und damit unter erhöhtem Druck durchzuführen. Schließlich-sind aus der **DE-C1 4207386** (Th.Goldschmidt) Betainkonzentrate mit Feststoffgehalten oberhalb von 40 Gew.-% bekannt, die 1 bis 3 Gew.-% freie Fettsäure und 0 bis 4 Gew.-% Glycerin enthalten, einen Gehalt an freiem Amidoamin von weniger als 1 Gew.-% und einen pH-Wert im Bereich von 5 bis 8 aufweisen.

Nachdem keines dieser Verfahren des Stands der Technik die kumulierte Aufgabenstellung zufriedenstellend zu lösen vermag, hat die Aufgabe der Erfindung darin bestanden, neue wäßrige Betaine zur Verfügung zu stellen, die fließ- und pumpfähig sind, auch bei längerer Lagerung nicht vergelen, einen Feststoffgehalt von mindestens 40 Gew.-% und einen minimierten Gehalt an unerwünschten Nebenbestandteilen, insbesondere chlorierten Stoffen und freien Aminen aufweisen.

### Beschreibung der Erfindung

Gegenstand der Erfindung sind niedrigviskose wäßrige Konzentrate von Betaintensiden der Formel (I), 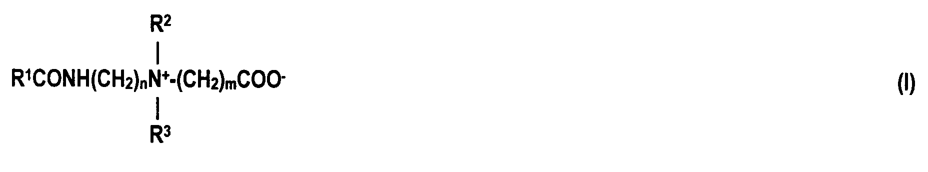 in der R¹CO für einen aliphatischen Acylrest mit 8 bis 22 Kohlenstoffatomen, R² und R³ unabhängig voneinander für einen Alkylrest mit 1 bis 4 Kohlenstoffatomen und n und m unabhängig voneinander für Zahlen im Bereich von 1 bis 5 steht, erhältlich durch Kondensation von Fettsäureaminoamiden mit Halogencarbonsäuresalzen in Gegenwart von Verflüssigungsmitteln, die sich dadurch auszeichnen, daß sie 1 bis 10 Gew.-% - bezogen auf die Konzentrate - Hydroxycarbonsäuren mit 2 bis 6 Kohlenstoffatomen aufweisen.

Überraschenderweise wurde gefunden, daß der Zusatz der kurzkettigen Hydroxycarbonsäuren die Viskosität der Konzentrate so weit herabsetzt, daß nunmehr fließ- und pumpfähige Produkte mit einem Feststoffgehalt im Bereich von 40 bis 55 Gew.-% zur Verfügung gestellt werden können, die auch bei längerer Lagerung keine Tendenz zur Vergelung zeigen. Die erfindungsgemäßen Konzentrate sind zudem hellfarbig und weisen einen minimierten Gehalt an chlorierten Verbindungen auf. Zur Sicherstellung einer ausreichend niedrigen Viskosität ist ein Gehalt an freiem Aminoamid nicht erforderlich. Zudem sind die Konzentrate auch ohne Zusatz von Konservierungsmitteln gegen antimikrobiellen Befall ausreichend geschützt.

### Betaine

Betaine, genauer Fettsäureamidobetaine, stellen bekannte Stoffe dar. Vorzugsweise bezieht sich die Erfindung auf wäßrige Konzentrate von Betaintensiden, die der Formel **(I)** folgen, in der R¹CO für einen Acylrest mit 8 bis 18 Kohlenstoffatomen, R¹ und R² für jeweils eine Methylgruppe und n und m unabhängig voneinander für 2 oder 3 steht.

### Hydroxycarbonsäuren

Im Sinne der Erfindung kommen als Hydroxycarbonsäuren vorzugsweise aliphatische Mono-, Di- und/oder Tricarbonsäuren mit 2 bis 6 Kohlenstoffatomen und 1, 2 oder 3 Hydroxylgruppen in Betracht. Typische Beispiele hierfür sind Glycolsäure, Milchsäure, Äpfelsäure, Weinsäure und Citronensäure, sowie deren Gemische. Die Hydroxycarbonsäuren werden vorzugsweise in Mengen von 1,5 bis 3,0 Gew.-% - bezogen auf die Konzentrate - eingesetzt. Obschon die verflüssigenden Hydroxycarbonsäuren den Konzentraten auch nachträglich zugesetzt werden können, hat es sich als vorteilhaft erwiesen, die Stoffe schon während der Kondensationsreaktion zuzugeben.

### Betainkonzentrate

Die erfindungsgemäßen Betainkonzentrate weisen einen Feststoffgehalt von mindestens 40, vorzugsweise von 40 bis 55 und insbesondere von 42 bis 48 Gew.-% - bezogen auf die Konzentrate - auf. Der Aktivsubstanzgehalt, d.h. der Gehalt an Betainen, liegt in der Regel 8 bis 12 Gew.-% niedriger. Im Hinblick auf die Lagerstabilität der Produkte hat es sich als vorteilhaft erweisen, die Konzentrate auf einen pH-Wert im Bereich von 5 bis 8 einzustellen. Der Gehalt an freiem Fettsäureaminoamid liegt üblicherweise unter 1 und insbesondere unter 0,5 Gew.-%, der Gehalt an Mono- bzw. Dichloressigsäure jeweils unter 5 ppm - bezogen auf die Konzentrate. In einer besonderen Ausführungsform des erfindungsgemäßen Verfahrens kann den Betainkonzentraten 0,1 bis 3 Gew.% frei C₁₂-C₁₈-Fettsäure und/oder 0,1 bis 3 Gew.-% eines Polyols, vorzugsweise Glycerin, Sorbitol oder Butylglucosid zugesetzt werden.

### Kondensationsreaktion

Ein weiterer Gegenstand der Erfindung betrifft ein Herstellung niedrigviskoser wäßriger Konzentrate von Betaintensiden, bei dem man Fettsäureaminoamide der Formel **(II)**, 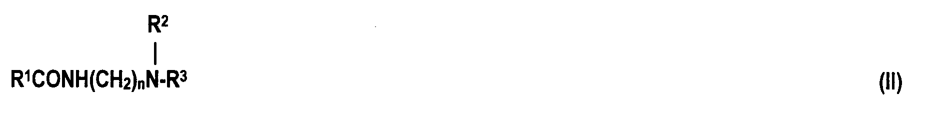 in der R¹CO für einen aliphatischen Acylrest mit 8 bis 22 Kohlenstoffatomen, R² und R³ unabhängig voneinander für einen Alkylrest mit 1 bis 4 Kohlenstoffatomen und n für Zahlen im Bereich von 1 bis 5 steht, mit Halogencarbonsäuren bzw. deren Salzen der Formel **(III)** kondensiert, 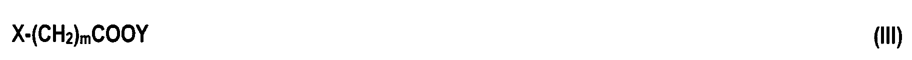 in der X für ein Halogen, Y für Wasserstoff oder ein Alkalimetall und m für Zahlen im Bereich von 1 bis 5 steht, und welches sich dadurch auszeichnet, daß man die Reaktion in Gegenwart von 1 bis 10 Gew.-% - bezogen auf die Konzentrate - Hydroxycarbonsäuren mit 2 bis 6 Kohlenstoffatomen als Verflüssigungsmitteln durchführt. Die Durchführung der Kondensationsreaktion erfolgt in an sich bekannter Weise, wobei man das Fettsäureaminoamid, vorzugsweise C_{12/18}- bzw. C_{8/18}-Kokosfettsäureaminoamide, und die Halogencarbonsäuren bzw. deren Salze, vorzugsweise Natriumchloracetat, unter Einhaltung eines pH-Wertes im Bereich von 7 bis 8 erhitzt und nachdem der Gehalt an freiem Aminoamid unter 0,5 Gew.-% abgesunken ist, die Reaktionsmischung in einem Druckgefäß über einen Zeitraum von 1 bis 2 h einer Nachbehandlung bei einer Temperatur von 100 bis 130°C und einem pH = 10 bis 14 unterwirft. Nach Abschluß der Nachreaktion empfiehlt es sich, das Konzentrat wieder auf einen neutralen pH-Wert einzustellen.

### Gewerbliche Anwendbarkeit

Die erfindungsgemäßen wäßrigen Betaine sind auch hochkonzentriert flüssig, lagerstabil und weisen einen minimierten Anteil an unerwünschten Nebenbestandteilen auf. Sie eignen sich für die Herstellung oberflächenaktiver Mittel, insbesondere von Reinigungsprodukten sowie Haarbehandlungs- und - pflegemitteln, in denen sie in Mengen von 1 bis 30, vorzugsweise 2 bis 10 Gew.-% - bezogen auf die Mittel - enthalten sein können. Ein weiterer Gegenstand der Erfindung betrifft daher die Verwendung von Hydroxycarbonsäuren mit 2 bis 6 Kohlenstoffatomen in Mengen von 1 bis 10 Gew.-% - bezogen auf die Konzentrate - als Verflüssigungsmittel zur Herstellung von niedrigviskosen wäßrigen Konzentraten von Betaintensiden.

### Beispiele

**Beispiel 1**. 227,2 g (1,91 mol) Natriumchloracetat wurden zusammen mit 91 g (1,2 mol) Glycolsäure, 55 g (0,27 mol) Laurinsäure und 54,5 g (0,59 mol) Glycerin in ca. 780 ml Wasser gelöst. Anschließend wurden bei 40°C 55,3 g (0,18 mol) eines Fettsäureaminoamids eingerührt, das durch Amidierung einer gehärteten Kokosfettsäure mit 3-N,N-Dimethylaminopropylamin hergestellt worden war und einen Gehalt von 4,6 Gew.-% titrierbaren Stickstoff aufwies. Nach Erwännen des Gemisches auf 90°C und Bildung einer klaren, dünnflüssigen Lösung wurden weitere 497,5 g (1,63 mol) des Fettsäureaminoamids zugegeben. Unter Einhaltung eines pH-Wertes zwischen 7,5 und 8,0 wurde die Reaktion fortgesetzt, bis nach ca. 2 h der Gehalt an freien Aminfunktionen unter 0,5 mmol/ 100 g, d.h. < 0,15 % Fettsäureaminoamid (ermittelt durch HPLC-Analyse) abgesunken war. Dem Ansatz wurden nun entsprechend einem pH-Wert von 12,5 in 10 Gew.-%iger Produktlösung 43,2 g 37 Gew.-%ige Natronlauge zugesetzt und das Produkt in einer Druckapparatur 1 h bei 120 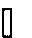 C gerührt. Nach der Abkühlung wurde der Produkt-pH-Wert mit 53,8 g 24-Gew.-%iger Salzsäure auf 7,0 eingestellt. Die Kenndaten des niedrigviskosen Produktes sind in Tabelle 1 zusammengefaßt. Die Viskosität wurde nach Brookfield bei 20°, 50 Upm, Spindel 3 bestimmt.

**Beispiel 2**. Beispiel 1 wurde unter Einsatz von 108 g (1,2 mol) Milchsäure wiederholt. Die Kenndaten des niedrigviskosen Produktes sind in Tabelle 1 zusammengefaßt.

**Vergleichbeispiel V1**. Beispiel 1 wurde ohne Zugabe von Glycolsäure wiederholt. Die Kenndaten des Produktes sind in Tabelle 1 zusammengefaßt.

**Vergleichbeispiel V2**. Beispiel 2 wurde ohne Zugabe von Milchsäure wiederholt. Die Kenndaten des Produktes sind in Tabelle 1 zusammengefaßt.

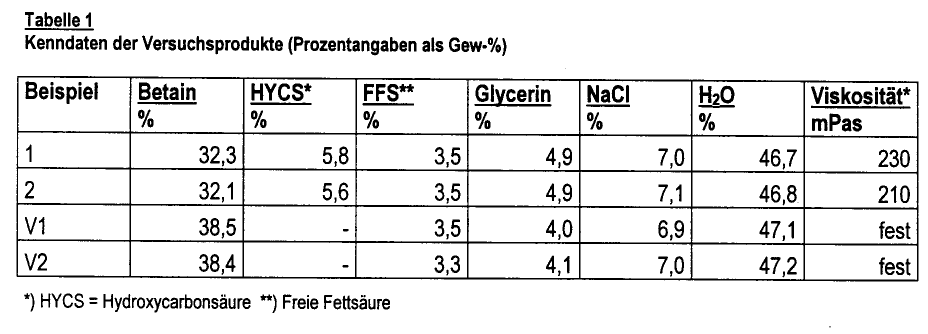

## Patentansprüche

1. Niedrigviskose wäßrige Konzentrate von Betaintensiden der Formel **(I)**, 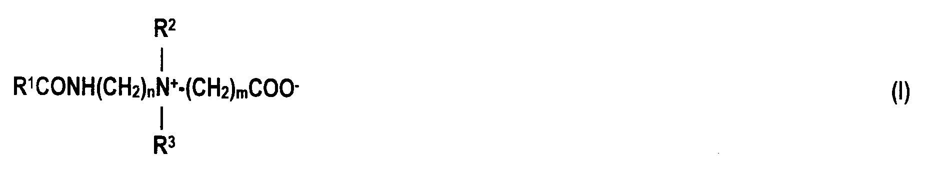 in der R¹CO für einen aliphatischen Acylrest mit 8 bis 22 Kohlenstoffatomen, R² und R³ unabhängig voneinander für einen Alkylrest mit 1 bis 4 Kohlenstoffatomen und n und m unabhängig voneinander für Zahlen im Bereich von 1 bis 5 steht, erhältlich durch Kondensation von Fettsäureaminoamiden mit Halogencarbonsäuresalzen in Gegenwart von Verflüssigungsmitteln, **dadurch gekennzeichnet,** daß sie 1 bis 10 Gew.-% - bezogen auf die Konzentrate - Hydroxycarbonsäuren mit 2 bis 6 Kohlenstoffatomen aufweisen.

2. Konzentrate nach Anspruch 1, **dadurch gekennzeichnet**, daß die Betaintenside der Formel **(I)** folgen, in der R¹CO für einen Acylrest mit 8 bis 18 Kohlenstoffatomen, R² und R³ für jeweils eine Methylgruppe und n und m unabhängig voneinander für 2 oder 3 stehen.

3. Konzentrate nach den Ansprüchen 1 und 2, **dadurch gekennzeichnet**, daß sie Hydroxycarbonsäuren enthalten, die ausgewählt sind aus der Gruppe, bestehend aus Glycolsäure, Milchsäure, Äpfelsäure, Weinsäure und/oder Citronensäure.

4. Konzentrate nach den Ansprüchen 1 bis 3, **dadurch gekennzeichnet**, daß der Gehalt an Hydroxycarbonsäuren im Bereich von 1,5 bis 3,0 Gew.-% - bezogen auf die Konzentrate - liegt.

5. Konzentrate nach den Ansprüchen 1 bis 4, **dadurch gekennzeichnet**, daß der Feststoffanteil im Bereich von 40 bis 55 Gew.-% - bezogen auf die Konzentrate - liegt.

6. Konzentrate nach den Ansprüchen 1 bis 5, **dadurch gekennzeichnet**, daß der pH-Wert im Bereich von 5 bis 8 liegt.

7. Konzentrate nach den Ansprüchen 1 bis 6, **dadurch gekennzeichnet**, daß der Gehalt an freiem Fettsäureaminoamid unter 1 Gew.-% - bezogen auf die Konzentrate - liegt.

8. Konzentrate nach den Ansprüchen 1 bis 7, **dadurch gekennzeichnet**, daß der Gehalt an Mono- bzw. Dichloressigsäure jeweils unter 5 ppm - bezogen auf die Konzentrate - liegt.

9. Verfahren zur Herstellung niedrigviskoser wäßriger Konzentrate von Betaintensiden, bei dem man Fettsäureaminoamide der Formel **(II)**, 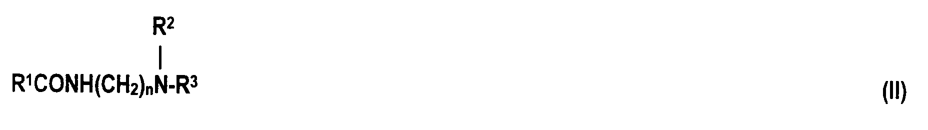 in der R¹CO für einen aliphatischen Acylrest mit 8 bis 22 Kohlenstoffatomen, R² und R³ unabhängig voneinander für einen Alkylrest mit 1 bis 4 Kohlenstoffatomen und n für Zahlen im Bereich von 1 bis 5 steht, mit Halogencarbonsäuren bzw. deren Salzen der Formel **(III)** kondensiert, 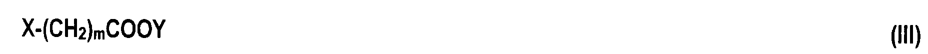 in der X für ein Halogen, Y für Wasserstoff oder ein Alkalimetall und m für Zahlen im Bereich von 1 bis 5 steht, **dadurch gekennzeichnet,** daß man die Reaktion in Gegenwart von 1 bis 10 Gew.-% - bezogen auf die Konzentrate - Hydroxycarbonsäuren mit 2 bis 6 Kohlenstoffatomen als Verflüssigungsmitteln durchführt.

10. Verwendung von Hydroxycarbonsäuren mit 2 bis 6 Kohlenstoffatomen in Mengen von 1 bis 10 Gew.-% - bezogen auf die Konzentrate - als Verflüssigungsmittel zur Herstellung von niedrigviskosen wäßrigen Konzentraten von Betaintensiden.

## Claims

1. Low-viscosity water-containing concentrates of betaine surfactants corresponding to formula **(I)**: 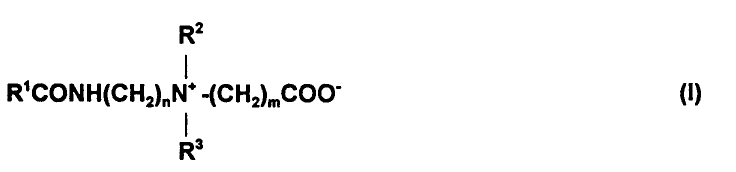 in which R¹CO is an aliphatic acyl group containing 8 to 22 carbon atoms, R² and R³ independently of one another represent an alkyl group containing 1 to 4 carbon atoms and n and m independently of one another are numbers of 1 to 5,
obtainable by condensation of fatty acid aminoamides with halocarboxylic acid salts in the presence of liquefying agents, characterized in that they contain 1 to 10% by weight - based on the concentrates - of hydroxycarboxylic acids containing 2 to 6 carbon atoms.

2. Concentrates as claimed in claim 1, characterized in that the betaine surfactants correspond to formula (I), in which R¹CO is an acyl group containing 8 to 18 carbon atoms, R² and R³ are each a methyl group and n and m independently of one another have a value of 2 or 3.

3. Concentrates as claimed in claims 1 and 2, characterized in that they contain hydroxycarboxylic acids selected from the group consisting of glycolic acid, lactic acid, malic acid, tartaric acid and/or citric acid.

4. Concentrates as claimed in claims 1 to 3, characterized in that the content of hydroxycarboxylic acids is in the range from 1.5 to 3.0% by weight, based on the concentrates.

5. Concentrates as claimed in claims 1 to 4, characterized in that they have a solids content of 40 to 55% by weight, based on the concentrates.

6. Concentrates as claimed in claims 1 to 5, characterized in that they have a pH value in the range from 5 to 8.

7. Concentrates as claimed in claims 1 to 6, characterized in that their content of free fatty acid aminoamide is below 1% by weight, based on the concentrates.

8. Concentrates as claimed in claims 1 to 7, characterized in that the contents of monochloroacetic acid and dichloroacetic acid are each below 5 ppm, based on the concentrates.

9. A process for the production of low-viscosity water-containing concentrates of betaine surfactants, in which fatty acid aminoamides corresponding to formula (II): 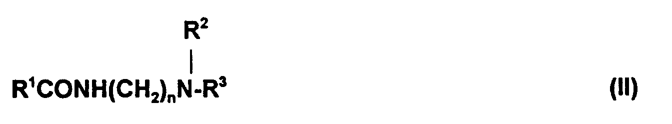 in which R¹CO is an aliphatic acyl group containing 8 to 22 carbon atoms, R² and R³ independently of one another represent an alkyl group containing 1 to 4 carbon atoms and n is a number of 1 to 5,
are condensed with halocarboxylic acids or salts thereof corresponding to formula **(III)**: 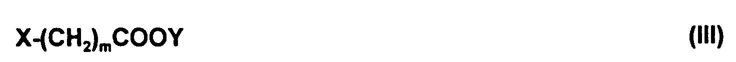 in which X is a halogen atom, Y is hydrogen or an alkali metal and m is a number of 1 to 5,
characterized in that the reaction is carried out in the presence of 1 to 10% by weight, based on the concentrates, of hydroxycarboxylic acids containing 2 to 6 carbon atoms as liquefying agents.

10. The use of hydroxycarboxylic acids containing 2 to 6 carbon atoms in quantities of 1 to 10% by weight, based on the concentrates, as liquefying agents for the production of low-viscosity water-containing concentrates of betaine surfactants.

## Revendications

1. Concentrés aqueux faiblement visqueux d'agents tensio-actifs bétaïniques de formule (I) 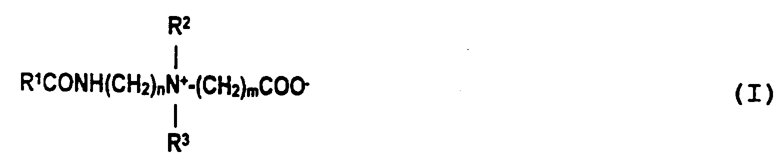 dans laquelle R¹CO représente un radical acyle ayant de 8 à 22 atomes de carbone, R² et R³ distinctement l'un de l'autre, représentent un radical acyle ayant de 1 à 4 atomes de carbone et n et m, distinctement l'un de l'autre, représentent des nombres dans la zone allant de 1 à 5 que l'on peut obtenir par condensation d'aminoamides d'acide gras avec des sels d'acide halogénocarboxylique en présence d'agents de fluidification,
caractérisés en ce qu'
ils contiennent de 1 à 10 % en poids, rapporté aux concentrés, d'acides hydroxycarboxyliques ayant de 2 à 6 atomes de carbone.

2. Concentrés selon la revendication 1,
caractérisé en ce que
les agents tensioactifs bétaïniques répondent à la formule (I) dans laquelle :
• R¹CO représente un radical acyle ayant de 8 à 18 atomes de carbone
• R² et R³ représentent respectivement un groupe méthyle
• et n et m distinctement l'un de l'autre représentent 2 ou 3

3. Concentrés selon les revendications 1 et 2,
caractérisés en ce qu'
ils renferment des acides hydroxycarboxyliques qui sont choisis dans le groupe constitué par l'acide glycolique, l'acide lactique, l'acide malique, l'acide tartrique et/ou l'acide citrique.

4. Concentrés selon les revendications 1 à 3,
caractérisés en ce que
la teneur en acides hydroxycarboxyliques se situe dans la plage allant de 1,5 à 3,0 % en poids, rapporté aux concentrés.

5. Concentrés selon les revendications 1 à 4,
caractérisés en ce que
la proportion de matières solides se situe dans la plage allant de 40 à 55 % en poids, rapporté aux concentrés.

6. Concentrés selon les revendication 1 à 5,
caractérisés en ce que
la valeur du pH se situe dans la plage allant de 5 à 8.

7. Concentrés selon les revendications 1 à 6,
caractérisés en ce que
la teneur en aminoamide d'acide gras se situe en dessous de 1 % en poids, rapporté aux concentrés.

8. Concentrés selon les revendications 1 à 7,
caractérisés en ce que
la teneur en acide mono ou dichloracétique se situe respectivement en dessous de 5 ppm, rapporté aux concentrés.

9. Procédé de fabrication de concentrés aqueux à faible viscosité d'agents tensioactifs bétaïniques, dans lequel on condense des aminoamides d'acides gras de formule (II) 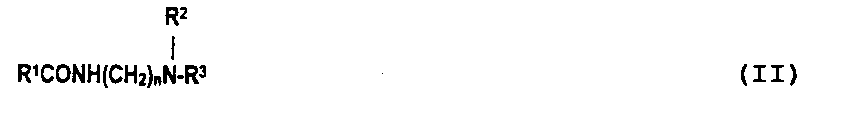 dans laquelle R¹CO représente un radical acyle aliphatique ayant de 8 à 22 atomes de carbone R² et R³ distinctement l'un et l'autre, représentent un radical alkyle ayant de 1 à 4 atomes de carbone
et n représente des nombres dans la zone allant de 1 à 5 avec des acides halogènocarboxyliques ou leurs sels de formule (III) 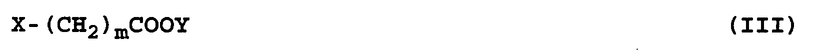 dans laquelle
• X représente un halogène
• Y représente de l'hydrogène ou un métal alcalin
• et m représente des nombres dans la zone allant de 1 à 5
caractérisé en ce qu'
on effectue la réaction en présence de 1 à 10 % en poids, rapporté aux concentrés, d'acides hydroxycarboxyliques ayant de 2 à 6 atomes de carbone en tant qu'agents fluidifiants.

10. Utilisation des acides hydroxycarboxyliques ayant de 2 à 6 atomes de carbone en quantités de 1 à 10 % en poids, rapporté aux concentrés, comme agent de fluidification en vue de la production de concentrés aqueux faiblement visqueux d'agents tensioactifs bétaïniques.
